(19) Europäisches Patentamt
European Patent Office
Office européen des brevets

(11) Veröffentlichungsnummer: **0 025 906**
B1

(12) **EUROPÄISCHE PATENTSCHRIFT**

(45) Veröffentlichungstag der Patentschrift: **27.10.82**

(51) Int. Cl.³: **C 07 C 118/00**, C 07 C 119/042

(21) Anmeldenummer: **80105229.1**

(22) Anmeldetag: **03.09.80**

(54) **1-Monohalogenierte Isocyanate und Verfahren zur Herstellung von Gemischen von 1-monohalogenierten Isocyanaten und 1,2-ungesättigten Isocyanaten.**

(30) Priorität: **13.09.79 DE 2937028**

(43) Veröffentlichungstag der Anmeldung: **01.04.81 Patentblatt 81/13**

(45) Bekanntmachung des Hinweises auf die Patenterteilung: **27.10.82 Patentblatt 82/43**

(84) Benannte Vertragsstaaten: **BE CH DE FR GB LI NL**

(56) Entgegenhaltungen: **DE-A-2 639 931**
**DE-A-2 705 695**

(73) Patentinhaber: **BASF Aktiengesellschaft, Carl-Bosch-Strasse 38, D-6700 Ludwigshafen (DE)**

(72) Erfinder: **Koenig, Karl-Heinz, Dr., Pierstrasse 8 A, D-6710 Frankenthal (DE)**
Erfinder: **Feuerherd, Karl-Heinz, Dr., Berner Weg 34, D-6700 Ludwigshafen (DE)**
Erfinder: **Oeser, Heinz-Guenter, Dr., Friedrich-Burschell-Weg 19, D-6700 Ludwigshafen (DE)**

EP 0 025 906 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 1-Monohalogenierte Isocyanate und Verfahren zur Herstellung von Gemischen von 1-monohalogenierten Isocyanaten und 1,2-ungesättigten Isocyanaten

Die Erfindung betrifft 1-monohalogenierte Isocyanate sowie ein Verfahren zur Herstellung von Gemischen von 1-monohalogenierten Isocyanaten und 1,2-ungesättigten Isocyanaten durch Umsetzung von 1-monohalogenierten Carbamidsäurehalogeniden oder Isocyanaten mit einem halogenfreien Isocyanat oder Diisocyanat oder durch Umsetzung von 1,2-ungesättigten Isocyanaten mit einem Carbamidsäurehalogenid oder Biscarbamidsäurehalogenid.

Die wirtschaftliche Synthese von 1-Alkenylisocyanaten ist wegen der großen Reaktivität dieser Verbindungen schwierig, da 1-Alkenylisocyanate nicht nur thermisch labil, sondern auch Säure-, basen- und hydrolyseempfindlich sind. Die bekanntesten Herstellungsmethoden sind die Curtius-Abbau von substituierten Acrylsäureaziden (J. Org. Chem., Band 26 [1961], Seiten 770 bis 779), die Vakuumpyrolyse von Trisvinylisocyanuraten (DAS 1 932 811) und die thermische Spaltung von N-tert.-Alkyl-N-(1-alkenyl)-carbamidsäurechloriden (DAS 1 922 412).

Es ist aus Liebigs Annalen der Chemie, Band 762 (1972), Seiten 88 bis 92 und Ber., Band 102 (1969), Seiten 2972 bis 2976 bekannt, das Ketimin des Diphenylketons und des tert.-Butylphenylketons mit Phosgen zu einem Gemisch von N-Chlorcarbonylaralkylketimen und α-Chloraralkylisocyanaten umzusetzen. Mehrfach halogensubstituierte 1-Halogenalkylisocyanate und 1-Phenyl-1-chloräthylisocyanat sind durch Halogenierung sowohl von unsubstituierten als auch bereits halogensubstituierten Alkylisocyanaten und Carbamidsäurehalogeniden zugänglich (US-Patent 3 437 680; DOS 1 418 666).

1-Fluorpropylisocyanat wird bei der Tieftemperatur-Fluorierung von Propylisocyanat neben anderen Reaktionsprodukten erhalten (J. Org. Chem., Band 32 [1967], Seiten 1633 bis 635).

Es ist aus J. Org. Chem., Band 28 (1963), Seiten 1825 bis 1830 bekannt, daß man Chlormethylisocyanat durch Umsetzung von Hydroxymethylisocyanat mit Thionylchlorid erhält. Entsprechend kann auch das 1,2,2,2-Tetrachloräthylisocyanat hergestellt werden.

Es ist aus der DE-OS 2 639 931 bekannt, daß man Carbamidsäurechloride mit organischen Mono- oder Polyisocyanaten, deren Siedepunkt über dem Siedepunkt des herzustellenden Monoisocyanats liegt, umsetzt und das gebildete Monoisocyanat durch Destillation oder mit einem Inertgasstrom abtrennt. Nach Anspruch und Beschreibung bleibt die am Stickstoffatom der Carbamidsäurechloride gebundene Kohlenstoffkette unverändert; Chlorwasserstoff wird lediglich an der Säurechlorid-Gruppierung abgespalten. Diese Lehre wird auch für den Fall der Verwendung durch Chloratome substituierter aliphatischer Reste gegeben. In der Beschreibung wird lediglich 2-Chloräthylcarbamidsäurechlorid als chlorierter Rest erwähnt.

In den Beispielen werden lediglich das Carbamidsäurechlorid des Methylisocyanats und als Reaktionspartner ein Diisocyanat gezeigt. 1-halogenierte Carbamidsäurechloride werden nicht beschrieben. Das höhersiedende Diisocyanat wird bei der Reaktion (Seite 13) in sein Carbamidsäurechlorid überführt.

Es wurde nun gefunden, daß man Gemische von 1-monohalogenierten Isocyanaten der Formel

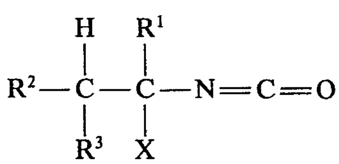

(Ia)

und 1,2-ungesättigten Isocyanaten der Formel

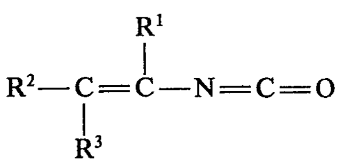

(Ib)

worin $R^1$, $R^2$ und $R^3$ gleich oder verschieden sein können und jeweils ein Wasserstoffatom, einen Alkylrest mit 1 bis 6 Kohlenstoffatomen oder einen Phenylrest bedeuten, das Restepaar $R^1$ und $R^2$ mit den benachbarten beiden Kohlenstoffatomen oder das Restepaar $R^2$ und $R^3$ mit dem benachbarten Kohlenstoffatom auch jeweils Glieder eines 5- oder 6-gliedrigen, alicyclischen Ringes bilden können, wobei $R^1$, $R^2$ und $R^3$ insgesamt bis zu 8 Kohlenstoffatomen enthalten können, X ein Chloratom oder ein Bromatom bezeichnet, vorteilhaft erhält, wenn man

a 1) 1-monohalogenierte Carbamidsäurehalogenide der Formel

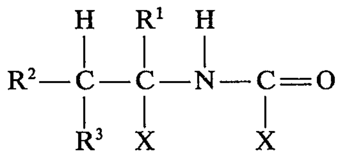

(II)

worin $R^1$, $R^2$, $R^3$ und X die vorgenannte Bedeutung besitzen, mit einem halogenfreien Isocyanat der Formel

$$R^4{-}N{=}C{=}O \qquad \text{(III)}$$

worin $R^4$ einen Alkylrest, Cycloalkylrest, Arylrest, Aralkylrest oder Alkylarylrest bedeutet, und/oder

a 2) mit einem Diisocyanat der Formel

$$O{=}C{=}N{-}R^5{-}N{=}C{=}O \qquad \text{(IV)}$$

worin $R^5$ einen Alkylenrest, Cycloalkylenrest, Arylenrest, Alkylarylenrest oder Arylalkylenrest bezeichnet, umsetzt oder

b 1) 1,2-ungesättigte Isocyanate I b mit einem 1-halogenfreien Carbamidsäurehalogenid der Formel

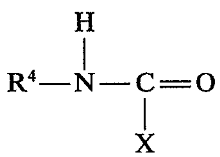

(V)

worin $R^4$ und X die vorgenannte Bedeutung besitzen, und/oder

b 2) mit einem Biscarbamidsäurehalogenid der Formel

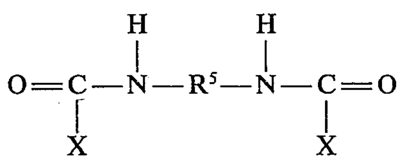

(VI)

worin $R^5$ und X die vorgenannte Bedeutung besitzen, und/oder

b3) mit einem 1-monohalogenierten Carbamidsäurehalogenid II umsetzt oder

c1) 1-monohalogenierte Isocyanate Ia mit einem halogenfreien Isocyanat III und/oder

c2) mit einem Diisocyanat IV

umsetzt.

Weiterhin wurden die neuen 1-monohalogenierten Isocyanate der Formel

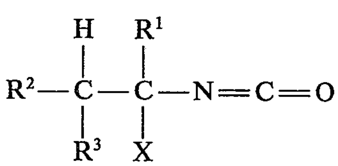

(Ia')

worin $R^1$, $R^2$ und $R^3$ gleich oder verschieden sein können und jeweils ein Wasserstoffatom, einen Alkylrest mit 1 bis 6 Kohlenstoffatomen oder einen Phenylrest bedeuten, das Restepaar $R^1$ und $R^2$ mit den benachbarten beiden Kohlenstoffatomen oder das Restepaar $R^2$ und $R^3$ mit dem benachbarten Kohlenstoffatom auch jeweils Glieder eines 5- oder 6-gliedrigen, alicyclischen Ringes bilden können, wobei $R^1$, $R^2$ und $R^3$ insgesamt bis zu 8 Kohlenstoffatomen enthalten können, X ein Chloratom oder Bromatom bezeichnet, wobei X nur das Bromatom bezeichnet, wenn $R^1$ für einen Phenylrest und gleichzeitig $R^2$ und $R^3$ jeweils für ein Wasserstoffatom stehen, gefunden.

Die Umsetzung kann für den Fall der Verwendung von 1-Chloräthylcarbamidsäurechlorid, Hexamethylendiisocyanat, Vinylisocyanat, 1-Chloräthylisocyanat, Äthylisocyanat durch die folgenden Formeln wiedergegeben werden:

a) 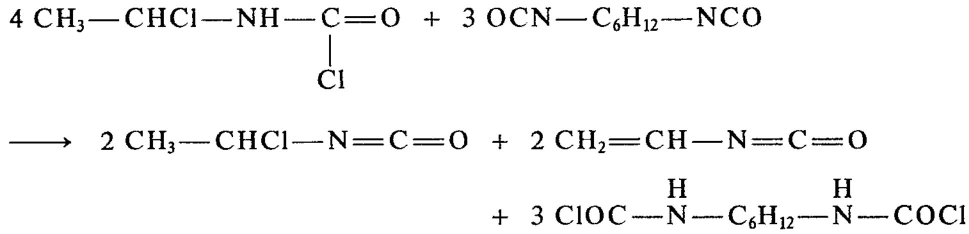

b) 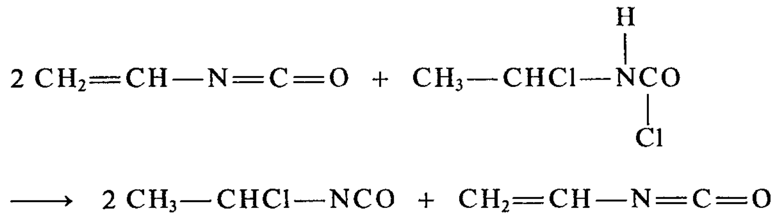

c) 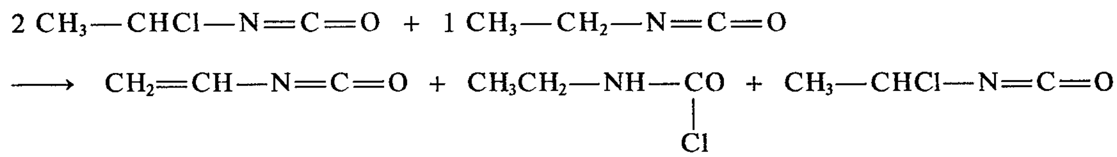

Im Hinblick auf den Stand der Technik liefert das Verfahren nach der Erfindung überraschend auf einfacherem und wirtschaftlicherem Wege 1,2-ungesättigte Isocyanate und 1-monohalogenierte Isocyanate in guter Ausbeute und Reinheit. Diese vorteilhaften Eigenschaften des erfindungsgemäßen Verfahrens waren nicht vorauszusehen, denn es ist bekannt, daß aliphatische Isocyanate mit Halogenwasserstoff bereits bei sehr niederen Temperaturen zu ihren entsprechenden Carbamidsäurehalogeniden reagieren. Diese spalten den Halogenwasserstoff reversibel unter langsamer Rückbildung des freien Isocyanats beim Erwärmen ab. Dieses Gleichgewicht läßt sich jedoch nicht ohne besondere Maßnahmen, insbesondere im Falle niedriger aliphatischer Isocyanate, zur Herstellung der freien Isocyanate ausnutzen, da der Halogenwasserstoff bei der Destillation zwar frei wird, jedoch sich wieder mit dem Isocyanat zum Carbamidsäurehalogenid zu rekombinieren sucht (Houben-Weyl, Methoden der Organischen Chemie, Band 8, Seiten 121, 124 und 131). Man hätte daher mit ähnlichen Folgereaktionen der Ausgangsstoffe und entsprechenden Gemischen mit hohen Anteilen der Ausgangsstoffe und ihren Nebenstoffen rechnen müssen. Es war ebenfalls nicht zu erwarten, daß man nach dem erfindungsgemäßen Verfahren auch Halogenwasserstoff, der nicht von der Carbamidsäurehalogenidgruppe, sondern von den Atomen des Kohlenstoffgerüsts stammt, unter Ausbildung einer olefinischen Doppelbindung eliminieren kann. Das erfindungsgemäße Verfahren ist umso überraschender, da 1-Alkenylisocyanate bekanntlich außerordentlich empfindlich gegen Halogenwasserstoff sind und schon bei Raumtemperatur mit diesem harzartigen Polymersubstanzen bilden (Recueil, Band 94 [1975], Seite 102) und schon bei sehr niederen Temperaturen diesen spontan addiert (DE-OS 27 32 284). In Anbetracht des Carbamidsäurechlorid-Isocyanat-Gleichgewichts (Houben-Weyl, Methoden der Organischen Chemie, Band 8, Seite 121) war zu erwarten gewesen, daß entstandenes 1-Alkenylisocyanat mit Halogenwasserstoff aus noch nicht umgesetztem 1-Halogenalkylcarbamidsäurehalogenid zu polymeren Verbindungen abreagieren würde. Weiterhin ist es überraschend, daß sich nach dem erfindungsgemäßen Verfahren neben 1-Alkenylisocyanaten auch die neuen 1-Halogenalkylisocyanate herstellen und durch Destillation abtrennen lassen.

Darüber hinaus muß die Halogenumlagerung bei 1-Halogenalkylisocyanaten entsprechend dem folgenden Gleichgewichtsschema in Betracht gezogen werden (Angewandte Chemie, Band 74 (1962), Seite 848).

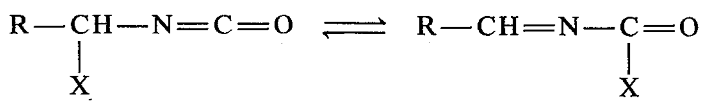

1-Halogenalkylisocyanate liegen demzufolge oft in der empfindlichen Halocarbonyliminform vor.

Es ist im Hinblick auf DE-OS 2 639 931 überraschend, daß sich im Falle a), b1) und b3) Gemische von 1-halogenierten und auch 1,2-ungesättigten Isocyanaten in guter Ausbeute bilden. Es konnte nicht erwartet werden, daß Halogenwasserstoff auch in 1,2-Stellung abgespalten wird.

Es war nicht vorhersehbar, daß das Isocyanat Ib zwar Halogenwasserstoff in 1,2-Stellung, aber nicht allein an der Isocyanatogruppierung anlagert. Überraschend im Hinblick auf diese Veröffentlichung erfolgt die Umsetzung somit in Abwesenheit von ungesättigten Carbamidsäurehalogeniden.

Bei der Herstellung werden Gemische von Endstoffen Ia und Ib, gebildet, deren Zusammensetzung

insbesondere von den gewählten Ausgangsstoffen und Mengenverhältnissen der Ausgangsstoffe bzw. Verfahren a) bis c), der Temperatur und der Abtrennung der Ausgangsstoffe aus dem Reaktionsgemisch abhängt. Man kann die Ausgangsstoffe II und III (Umsetzung a1) oder die Ausgangsstoffe II und IV (a2) oder die Stoffe Ib und V (b1) oder die Stoffe Ib und VI (b2) oder die Stoffe Ib und II (b3) oder die Stoffe Ia und III (c1) oder die Stoffe Ia und IV (c2) jeweils miteinander in stöchiometrischer Menge oder im Überschuß jeder Komponente zur anderen umsetzen, zweckmäßig im Falle des Verfahrens a1) von 15 bis 25 Mol Stoff III je Mol Stoff II, im Falle des Verfahrens a2) von 3 bis 14 Mol Stoff IV je Mol Stoff II, im Falle des Verfahrens b1) von 0,5 bis 1 Mol Stoff V je Mol Stoff Ib, im Falle des Verfahrens b2) von 0,25 bis 0,5 Mol Stoff VI je Mol Stoff Ib, im Falle des Verfahrens b3) von 0,5 bis 1 Mol Stoff II je Mol Stoff Ib, im Falle des Verfahrens c1) von 7 bis 13 Mol Stoff III je Mol Stoff Ia, im Falle des Verfahrens c2) von 1 bis 8 Mol Stoff IV je Mol Stoff Ia. Man erhält im allgemeinen im Falle der erfindungsgemäßen Umsetzung a1) Gemische von 0,05 bis 0,2 Mol Endstoff Ia je Mol Endstoff Ib, bei Verwendung vorgenannter zweckmäßiger Mengen an Ausgangsstoffen von 0,05 bis 0,1 Mol Endstoff Ia je Mol Endstoff Ib, im Falle der Umsetzung a2) Gemische von 0,2 bis 0,7 Mol Endstoff Ia je Mol Endstoff Ib, bei Verwendung vorgenannter zweckmäßiger Mengen an Ausgangsstoffen von 0,3 bis 0,6 Mol Endstoff Ia je Mol Endstoff Ib, im Falle der Umsetzung b1) Gemische von 0,1 bis 1 Mol Endstoff Ia je Mol Endstoff Ib, bei Verwendung vorgenannter zweckmäßiger Mengen an Ausgangsstoffen von 0,1 bis 0,5 Mol Endstoff Ia je Mol Endstoff Ib, im Falle der Umsetzung b2) Gemische von 0,1 bis 1 Mol Endstoff Ia je Mol Endstoff Ib, bei Verwendung vorgenannter zweckmäßiger Mengen an Ausgangsstoffen von 0,1 bis 0,5 Mol Endstoff Ia je Mol Endstoff Ib, im Falle der Umsetzung b3) Gemische von 2 bis 20 Mol Endstoff Ia je Mol Endstoff Ib, bei Verwendung vorgenannter zweckmäßiger Mengen an Ausgangsstoffen von 7 bis 20 Mol Endstoff Ia je Mol Endstoff Ib, im Falle der Umsetzung c1) Gemische von 0,1 bis 0,5 Mol Endstoff Ia je Mol Endstoff Ib, bei Verwendung vorgenannter zweckmäßiger Mengen an Ausgangsstoffen von 0,1 bis 0,2 Mol Endstoff Ia je Mol Endstoff Ib, im Falle im Gemisch bei Verfahren c2) Gemische von 0,1 bis 0,4 Mol Endstoff Ia je Mol Endstoff Ib, bei Verwendung vorgenannter zweckmäßiger Mengen an Ausgangsstoffen von 0,1 bis 0,2 Mol Endstoff Ia je Mol Endstoff Ib. Je höher man die Konzentration an den Stoffen a1) III, a2) IV, b1) V, b2) VI, b3) II, c1) III, c2) IV im Vergleich zum anderen jeweiligen Ausgangsstoff wählt, desto größer wird der Anteil an Endstoff Ib im Gemisch im Falle des Verfahrens a1) und entsprechend von Ib im Gemisch bei Verfahren a2), Ia im Gemisch bei Verfahren b1), Ia im Gemisch bei Verfahren b2), Ia im Gemisch bei Verfahren b3), Ib im Gemisch bei Verfahren c1), Ib im Gemisch bei Verfahren c2). Durch einen entsprechenden Versuch kann man somit leicht für jedes Verfahren ein gewünschtes Mengenverhältnis der Endstoffe Ia und Ib bzw. die wesentliche Bildung nur einer Komponente im Endstoffgemisch festlegen. Im Falle des Verfahrens b3) erhält man im wesentlichen nur den Endstoff Ia, im Falle des Verfahrens a1) und a2) im wesentlichen nur den Endstoff Ib.

Bevorzugte Ausgangsstoffe II; III; IV; V; VI und demzufolge bevorzugte Endstoffe Ia und Ib sind solche, in deren Formeln die Reste $R^1$, $R^2$ und $R^3$ gleich oder verschieden sein können und die vorgenannte Bedeutung besitzen, wobei die Bedeutung des Alkylrestes insbesondere mit ein oder 2 Kohlenstoffatomen vorteilhaft ist, $R^4$ einen Alkylrest mit 1 bis 12, insbesondere 1 bis 6 Kohlenstoffatomen, einen Cyclohexylrest, einen Aralkylrest oder Alkylarylrest mit 7 bis 12 Kohlenstoffatomen, oder einen Phenylrest bezeichnet, $R^5$ für einen Alkylenrest mit 1 bis 12 Kohlenstoffatomen, insbesondere 1 bis 6 Kohlenstoffatomen, einen Cyclohexylenrest, einen Aralkylenrest oder Alkylarylenrest mit 7 bis 12 Kohlenstoffatomen oder einen Phenylenrest steht, X ein Bromatom oder insbesondere ein Chloratom bezeichnet. $R^4$ als Cycloalkylrest oder $R^5$ als Cycloalkylenrest können zweckmäßig einen mono- oder bicyclischen, gegebenenfalls durch Alkylgruppen mit 1 bis 3 Kohlenstoffatomen substituierten Rest bezeichnen; im Falle eines bicyclischen Restes können die beiden Cycloalkylkerne anelliert oder direkt oder über eine Methylengruppe miteinander verbunden sein, die beiden Isocyanatogruppen im Falle der Ausgangsstoffe IV können 1) beide an einem Cycloalkylkern oder 2) an einem der beiden Cycloalkylkerne oder 3) jeweils nur eine Gruppe an einem der beiden Cycloalkylreste und die andere Isocyanatogruppe an dem anderen der beiden Cycloalkylreste liegen oder 4) ein oder beide Isocyanatogruppen sind über Alkylengruppen mit 1 bis 3 Kohlenstoffatomen mit einem oder beiden Cycloalkylkernen verbunden. Die Ausgangsstoffe II werden auf einfache Weise, z. B. nach dem in der DOS 2 741 980 beschriebenen Verfahren, hergestellt. Die nach der Reaktion durch z. B. Destillation aus dem Gemisch abgetrennten Endstoffe Ia können als Ausgangsstoffe bei den Verfahren c1) oder c2) und entsprechend die Endstoffe Ib bei den Verfahren b1), b2), b3) erneut wieder für die erfindungsgemäße Umsetzung zur Erzielung eines hohen Anteils an einem oder anderen Endstoff verwendet werden. Gegebenenfalls kann man Ausgangsstoff II auch mit einem Gemisch der Ausgangsstoffe III und IV, Isocyanat Ib mit einem Gemisch von Ausgangsstoff V, VI oder II, VI oder II, V oder II, V, Vi, Isocyanat Ia mit einem Gemisch von Ausgangsstoff III und IV umsetzen.

Geeignete Ausgangsstoffe II sind beispielsweise: 1-Chloräthylcarbamidsäurechlorid, 1-Bromäthylcarbamidsäurebromid, 1-Chlorpropylcarbamidsäurechlorid, 1-Brompropylcarbamidsäurebromid, 1-Chlor-1-methyläthylcarbamidsäurechlorid, 1-Brom-1-methyläthylcarbamidsäurebromid, 2-Chlorbutyl-(2)-carbamidsäurechlorid, 2-Brombutyl-(2)-carbamidsäurebromid, 1-Chlor-2-methylpropylcarbamidsäurechlorid, 1-Brom-2-methylpropylcarbamidsäurebromid, 2-Chlor-3-methylbutyl-(2)-carbamidsäurechlorid, 2-Brom-3-methylbutyl-(2)-carbamidsäurebromid, 2-Phenyl-1-chloräthylcarbamidsäurechlorid, 1-Chlor-cyclohexyl-(1)-carbamidsäurechlorid, 1-Chlor-1-cyclohexyläthyl-carbamidsäurechlorid.

Geeignete Ausgangsstoffe III und V sind beispielsweise: Äthylisocyanat, Propylisocyanat, Isopropylisocyanat, Butylisocyanat, Isobutylisocyanat, sek.-Butylisocyanat, tert.-Butylisocyanat, Pentylisocyanat, 3-Methyl-butyl-isocyanat, Hexylisocyanat, 2-Äthylhexylisocyanat, Cyclohexylisocyanat, Phenylisocyanat, Benzylisocyanat, 3-Methylphenylisocyanat, $\alpha$-Naphthylisocyanat; entsprechende Carbamidsäurechloride und Carbamidsäurebromide vorgenannter Monoisocyanate III.

Als Ausgangsstoffe IV und VI kommen z. B in Frage: Hexamethylendiisocyanat, Toluylendiisocyanat, Bis-(3-methyl-4-isocyanato-cyclohexyl)-methan, 1,1,4,4-Tetramethylbutandiisocyanat(1,4), 1,1,6,6-Tetramethylhexandiisocyanat(1,6), 3-Isocyanato-methyl-3,5,5-trimethylcyclohexylisocyanat; entsprechende Biscarbamidsäurechloride und Biscarbamidsäurebromide vorgenannter Diisocyanate IV.

Für die Verfahren b1) bis b3) bzw. c1) bis c2) kommen beispielsweise die den vorgenannten, als geeignet aufgeführten Carbamidsäurehalogeniden II entsprechenden Isocyanate Ia oder Ib in Betracht.

Vorteilhaft für die Auswahl der Ausgangsstoffe II bis VI ist der bei einer erfindungsgemäßen Umsetzung zu erwartende Siedepunkt des 1,2-ungesättigten Isocyanats Ib. Der Siedepunkt dieses Isocyanats sollte geringfügig, vorzugsweise jedoch deutlich niedriger, vorteilhaft 10 bis 100°C niedriger, als der der Ausgangsstoffe II bis VI liegen. Ebenso sollte der Siedepunkt des 1-Halogenalkylisocyanats möglichst tiefer, vorteilhaft 10 bis 50°C tiefer, als der der Ausgangsstoffe II bis VI liegen. Andernfalls kann aber beim Abdestillieren eines Gemisches aus zwei oder drei Komponenten, z. B. der Isocyanate Ia, Ib und III, der Anteil an Endstoff Ia durch erneute Destillation des Ausgangsstoffs III fast vollkommen in das erwünschte Isocyanat Ib überführt werden. Bevorzugt für die Durchführung des erfindungsgemäßen Verfahrens ist die sofortige und kontinuierliche Entfernung des gewünschten Gemisches oder einer der Endstoffe, vorzugsweise des Endstoffes Ib, aus dem Gleichgewicht mit Hilfe eines Inertgasstroms bei Normaldruck, besonders bei niedersiedenden Reaktionsprodukten oder durch Reaktionsführung im Vakuum oder auch durch Kombination von Inertgasstrom und die Arbeitsweise im Vakuum, vorzugsweise jedoch durch einen Inertgasstrom, z. B. trockenen Stickstoff oder trockene Luft.

Ist in einem Fall der Verfahrensweise a1) oder a2) eine eindeutige Abtrennung des Isocyanats Ib bei der einmaligen Umsetzung von 1-Halogencarbamidsäurehalogeniden II nicht zu erzielen, so kann zweckmäßig das mit dem Trägergasstrom ausgetragene rohe Endstoffgemisch erneut mit Ausgangsstoff III oder IV zum gewünschten 1,2-ungesättigen Isocyanat umgesetzt werden. Der Reaktionsrückstand, der überwiegend aus dem Carbamidsäurechlorid der Ausgangsstoffe III oder IV besteht, kann in bekannter Weise zum Isocyanat regeneriert werden, z. B. durch thermische Halogenwasserstoffabspaltung und anschließende Destillation des rohen Isocyanats, wobei der freiwerdende Halogenwasserstoff anderweitig zur Herstellung von z. B. wäßrigen Halogenwasserstoffsäure Verwendung finden kann, d. h. der gesamte abgespaltene Halogenwasserstoff ist wirtschaftlich wieder nutzbar und recyclisierbar. Meist ist die Isolierung reiner 1,2-ungesättigter Isocyanate Ib möglich, wohingegen 1-Halogenisocyanate Ia bei Raumtemperatur meist nur in 70- bis 90gewichtsprozentiger Reinheit zu erhalten sind, weil 1-Halogenisocyanate schon bei Raumtemperatur überraschenderweise im Gleichgewicht mit den korrespondierenden 1-Alkenylisocyanaten vorliegen.

Je höher die Reaktionstemperatur und je länger die Reaktionszeit, desto höher ist der Anteil von Endstoff Ib im Endgemisch. Die Umsetzung wird in der Regel bei einer Temperatur von 0 bis 150°C, im Falle der Herstellung von Gemischen mit mehr als 1,5, insbesondere oberhalb 1,5 bis 20 Mol Endstoff Ia je Mol Endstoff Ib, zweckmäßig 0 bis 50°C, im Falle der Herstellung von Gemischen mit 0,5 bis 1,5, insbesondere 0,9 bis 1,1 Mol Endstoff Ia je Mol Ib, zweckmäßig 40 bis 70°C, im Falle der Herstellung von Gemischen mit weniger als 0,5, insbesondere 0,05 bis unterhalb 0,5 Mol Endstoff Ia je Mol Endstoff Ib, zweckmäßig 70 bis 120°C, drucklos oder unter Druck, kontinuierlich oder diskontinuierlich durchgeführt. Vorteilhaft beginnt man die Umsetzung bei einer Temperatur von 0 bis 30°C, erhöht langsam die Temperatur und beendet die Reaktion bei den vorgenannten, zweckmäßigen Reaktionstemperaturen. Die Reaktionszeit beträgt im allgemeinen 0,1 bis 5 Stunden, vorteilhaft im Falle der Herstellung von Gemischen mit mehr als 1,5, insbesondere oberhalb 1,5 bis 20 Mol Endstoff Ia je Mol Endstoff Ib 2 bis 4 Stunden, im Falle der Herstellung von Gemischen mit 0,5 bis 1,5, insbesondere 0,9 bis 1,1 Mol Endstoff Ia je Mol. Endstoff Ib 0,5 bis 3 Stunden im Falle der Herstellung von Gemischen mit weniger als 0,5, insbesondere 0,05 bis unterhalb 0,5 Mol Endstoffe Ia je Mol Endstoff Ib 1,5 bis 3 Stunden. Bereits bei niederen Temperaturen um +10°C setzt die Umwandlung der Carbamidsäurehalogenidgruppe des 1-Halogencarbamidsäurehalogenids II zur Isocyanatgruppe ein, so daß mit Hilfe eines geeignet starken Inertgasstromes der entstehende Gleichgewichtsanteil an 1-Halogenisocyanat Ia ausgetragen werden kann, während bei höheren Temperaturen im Bereich ab 30°C auch mit dem vermehrten Auftreten der zugehörigen 1,2-ungesättigten Isocyanate gerechnet werden muß.

In einer vorteilhaften Ausführungsform stellt man im wesentlichen Endstoff Ib her, in den man die Reaktion a1) oder a2) bei 0 bis 50°C beginnt und bei einer Temperatur von oberhalb 50 bis 150°C

beendet und gleichzeitig während der Reaktion des gebildeten Endstoffs Ib abtrennt. Die Abtrennung erfolgt zweckmäßig durch gleichzeitige fraktionierte Destillation und/oder Druchleiten von Inertgas, z. B. Stickstoff.

Bevorzugt setzt man in Abwesenheit von weiteren Lösungsmitteln um, jedoch können unter den Reaktionsbedingungen inerte Lösungsmittel verwendet werden. Als Lösungsmittel kommen z. B. in Frage: aromatische Kohlenwasserstoffe, z. B. Toluol, Äthylbenzol, o-, m-, p-Xylol, Isopropylbenzol, Methylnaphthalin; Halogenkohlenwasserstoffe, insbesondere Chlorkohlenwasserstoffe, z. B. Tetrachloräthylen, 1,1,2,2- oder 1,1,1,2-Tetrachloräthan, Dichlorpropan, Methylenchlorid, Dichlorbutan, Isopropylbromid, n-Propylbromid, Butylbromid, Chloroform, Äthyljodid, Propyljodid, Tetrachlorkohlenstoff, 1,1,1- oder 1,1,2-Trichloräthan, Trichloräthylen; aliphatische oder cycloaliphatische Kohlenwasserstoffe, z. B. Haptan, Nonan, Benzinfraktionen innerhalb eines Siedepunktintervalls von 70 bis 190°C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan; und entsprechende Gemische. Zweckmäßig verwendet man das Lösungsmittel in einer Menge von 200 bis 10 000 Gewichtsprozent, vorzugsweise von 300 bis 2000 Gewichtsprozent, bezogen auf Ausgangsstoff II bzw. im Falle des Verfahrens b Stoff Ia oder im Falle des Verfahrens c Stoff Ib.

Die Reaktion kann wie folgt durchgeführt werden: Ein Gemisch der Ausgangsstoffe wird bei der Reaktionstemperatur während vorgenannter Reaktionszeit umgesetzt. Aus dem Reaktionsgemisch werden dann die Endstoffe Ia und Ib, zweckmäßig direkt während oder nach der Reaktion durch Erhöhung der Temperatur und fraktionierte Destillation, abgetrennt. Anstelle der reinen 1-Halogencarbamidsäurehalogenide II lassen sich auch die Reaktionsgemische aus der Herstellung dieser Ausgangsstoffe, z. B. die rohen Halogenierungsgemische verwenden, die bei der Halogenierung von Alkylisocyanaten bzw. deren Carbamidsäurehalogenide anfallen.

Die erfindungsgemäß herstellbaren 1,2-ungesättigten Isocyanate Ib und 1-monohalogenierten Isocyanate Ia sind wertvolle Ausgangsstoffe für die Herstellung von Schädlingsbekämpfungsmitteln, Farbstoffe, Pharmazeutika, Textilhydrophobierungsmitteln, Waschmitteln, Kunststoffen, Bleichmitteln und Klebstoffen, da sie neben einer reaktiven Isocyanatgruppe noch eine aktivierte Doppelbindung bzw. ein aktiviertes $\alpha$-C-Atom besitzen. Zudem sind 1-Alkenylisocyanate wichtige Monomere, die in vielfältiger Weise zu Ketten- und Leiterpolymeren, wie z. B. strahlungshärtenden Lackharzen, umgesetzt werden können (Chem. High Polymers [Tokyo] 13 [1956], Seite 390; J. Polymer Sc. 35 [1959], Seite 215, J. Org. Chem. 26 [1961], Seite 770; J. of Coatings Techn. 49 [1977], Seite 82). Sie können zu Urethanen, z. B. für die Verwendung als Schaumstoffe oder hochmolekulare Überzüge mit hoher Flexibilität, oder Harnstoffen umgesetzt werden. Bezüglich der Verwendung wird auf vorgenannte Veröffentlichungen und Ullmanns Encyklopädie der technischen Chemie, Band 9, Seiten 11, 12, 404 sowie Band 17, Seite 204 (3. Auflage), hingewiesen.

Die in den folgenden Beispielen aufgeführten Teile bedeuten Gewichtsteile. Sie verhalten sich zu den Volumenteilen wie Kilogramm zu Liter.

## Beispiel 1

Eine Lösung von 57 Teilen 1-Chloräthylcarbamidsäurechlorid in 100 Volumenteile Hexamethylendiisocyanat wird langsam bei 100°C während 0,5 Stunden unter die Oberfläche von 572 Teilen Hexamethylendiisocyanat zugegeben. Gleichzeitig bläst man Stickstoff durch die Lösung und kühlt das Abgas mittels einer Kühlfalle auf −100°C. Man destilliert das Reaktionsgemisch fraktionierend. Man erhält 10,2 Teile (36,8% der Theorie) Vinylisocyanat vom Kp (1013 mbar) 38,5°C und 6,8 Teile (16,1% der Theorie) 1-Chloräthylisocyanat vom Kp (101,3 mbar) 92°C.

## Beispiel 2

30 Teile 1-Chloräthylcarbamidsäurechlorid werden in 100 Volumenteilen 1-Chlornaphthalin gelöst und dem Gemisch während 0,3 Stunden bei 2°C eine Lösung von 16 Teilen Vinylisocyanat in 20 Volumenteilen 1-Chlornaphthalin zugegeben.

Man erhält 12,5 Teile (8% der Theorie) Vinylisocyanat und 43 Teile (92% der Theorie) 1-Chloräthylisocyanat (gaschromatographisch geprüft).

## Beispiel 3

48,5 Teile 1-Bromäthylcarbamidsäurebromid werden in 100 Volumenteilen $\alpha$-Chlornaphthalin gelöst und dem Gemisch bei 2°C während 0,4 Stunden eine Lösung von 16 Teilen Vinylisocyanat in 20 Volumenteilen 1-Chlornaphthalin zugegeben. Das Gemisch wird 2 Stunden bei 24°C gehalten. Man erhält 3,6 Teile (11% der Theorie) Vinylisocyanat und 58,5 Teile (88% der Theorie) 1-Bromäthylisocyanat (gaschromatographisch geprüft).

Beispiel 4

Man trägt bei 22° C in 700 Volumenteile Isopropylisocyanat 57 Teile 1-Chloräthylcarbamidsäurechlorid ein, erwärmt unter Druchleiten eines schwachen Stickstoffstromes in 1,5 Stunden bis auf 70° C und kondensiert das Reaktionsgemisch bei −70° C in einer Kühlfalle. Man erhält 12 Teile (43% der Theorie) Vinylisocyanat vom Kp (1013 mbar) 38,5° C, 0,85 Teile (2% der Theorie) Chloräthylisocyanat vom Kp (1013 mbar) 92° C und 218 Teile Isopropylisocyanat.

Beispiel 5

Man löst 57 Teile 1-Chloräthylcarbamidsäurechlorid in 520 Teilen Äthylisocyanat bei 22° C und bläst trockenen Stickstoff durch die Lösung, wobei man eine Raschigringkolonne verwendet, um eine Vortrennung des 3 Komponentengemisches in der Kolonne (Äthyl-, Vinyl- und 1-Chloräthylisocyanat) zu erzielen. Man destilliert das Reaktionsgemisch fraktionierend während 3 Stunden. Man erhält 14 Teile (50,5% der Theorie) Vinylisocyanat vom Kp (1013 mbar) 38,5° C und 466 Teile Äthylisocyanat vom Kp (1013 mbar) 60° C.

Beispiel 6

563 Teile 1-Chloräthylcarbamidsäurechlorid werden in 2800 Volumenteilen Hexamethylendiisocyanat gelöst. Die Lösung von insgesamt 3100 Volumenteilen wird in 3 Teilen (500; 1500; 1100 Volumenteilen) mit unterschiedlicher Verweilzeit (150; 310; 225 Minuten) in einen Dünnfilmverdampfer gegeben, der bei Normaldruck mit einem Stickstoffgegenstrom betrieben wird. Die Manteltemperatur beträgt 73 bis 75° C, die Übergangstemperatur 48 bis 54° C. Das Reaktionsgemisch wird in einer Vorlage und zwei nachgeschalteten Kühlfallen aufgefangen und der Gehalt gaschromatographisch ermittelt. Man erhält 145,3 Teile (53% der Theorie) Vinylisocyanat vom Kp (1013 mbar) 38,5° C und 73,8 Teile (17,7% der Theorie) 1-Chloräthylisocyanat vom Kp (1013 mbar) 92° C.

Beispiel 7

Ein Gemisch aus 57 Teilen 1-Chloräthylcarbamidsäurechlorid, 156 Teilen Äthylisocyanat und 841 Teilen Hexamethylendiisocyanat wird unter Durchleiten eines Stickstoffstromes von 22° C in 3 Stunden auf 90° C erwärmt. Man erhält in der Destillationsvorlage ein Gemisch aus 11,8 Teilen (42,5% der Theorie) Vinylisocyanat vom Kp (1013 mbar) 38,5° C, 10,1 Teilen (23,8% der Theorie) 1-Chloräthylisocyanat vom Kp (1013 mbar) 92° C und 149 Teilen Äthylisocyanat.

Beispiel 8

Zu 33 Teilen Äthylcarbamidsäurechlorid (gelöst in 100 Volumenteilen 1-Chlornaphthalin) gibt man bei 2° C 21 Teile Vinylisocyanat. Bei langsamer Temperatursteigerung des Gemisches verfolgt man dann gaschromatographisch die Verschiebung des Gleichgewichtes im Reaktionsgemisch (Tabelle) ohne Einleiten von Stickstoff. Äthylcarbamidsäurechlorid ist schon bei Raumtemperatur fast völlig umgesetzt und nicht mehr nachweisbar.

| Temperatur | Ausbeute in % der Theorie | | |
|---|---|---|---|
| °C | Vinyl-isocyanat | Äthyl-isocyanat | $\alpha$-Chloräthyl-isocyanat |
| 20 | 29 | 48 | 22 |
| 40 | 19 | 47 | 33 |
| 50 | 9,7 | 49,6 | 40,7 |

**Patentansprüche**

1. Verfahren zur Herstellung eines Gemisches von 1-monohalogenierten Isocyanaten der Formel

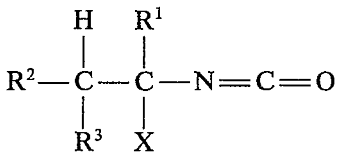

(Ia)

und 1,2-ungesättigten Isocyanaten der Formel

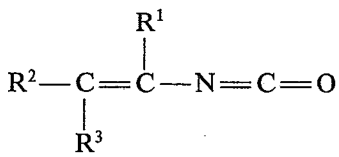

(Ib)

worin $R^1$, $R^2$ und $R^3$ gleich oder verschieden sein können und jeweils ein Wasserstoffatom, einen Alkylrest mit 1 bis 6 Kohlenstoffatomen oder einen Phenylrest bedeuten, das Restepaar $R^1$ und $R^2$ mit den benachbarten beiden Kohlenstoffatomen oder das Restepaar $R^2$ und $R^3$ mit dem benachbarten Kohlenstoffatom auch jeweils Glieder eines 5- oder 6gliedrigen, alicyclischen Ringes bilden können, wobei $R^1$, $R^2$ und $R^3$ insgesamt bis zu 8 Kohlenstoffatomen enthalten können, X ein Chlor- oder Bromatom bezeichnet, dadurch gekennzeichnet, daß man

a 1) 1-monohalogenierte Carbamidsäurehalogenide der Formel

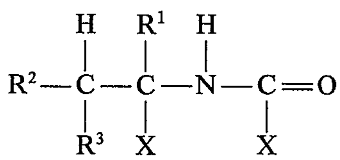

(II)

worin $R^1$, $R^2$, $R^3$ und X die vorgenannte Bedeutung besitzen, mit einem halogenfreien Isocyanat der Formel

$$R^4—N=C=O \qquad (III)$$

worin $R^4$ einen Alkylrest, Cycloalkylrest, Arylrest, Aralkylrest oder Alkylarylrest bedeutet, und/oder

a 2) mit einem Diisocyanat der Formel

$$O=C=N—R^5—N=C=O \qquad (IV)$$

worin $R^5$ einen Alkylenrest, Cycloalkylenrest, Arylenrest, Alkylarylenrest oder Arylalkylenrest bezeichnet, umsetzt oder

b 1) 1,2-ungesättigte Isocyanate I b mit einem 1-halogenfreien Carbamidsäurehalogenid der Formel

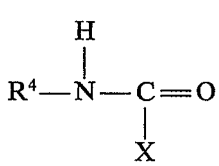

(V)

worin $R^4$ und X die vorgenannte Bedeutung besitzen, und/oder

b 2) mit einem Biscarbamidsäurehalogenid der Formel

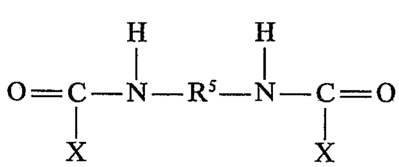

(VI)

worin $R^5$ und X die vorgenannte Bedeutung besitzen, und/oder

b3) mit einem 1-monohalogenierten Carbamidsäurehalogenid II umsetzt oder
c1) 1-monohalogenierte Isocyanate Ia mit einem halogenfreien Isocyanat III und/oder
c2) mit einem Diisocyanat IV

umsetzt.

2. 1-monohalogenierte Isocyanate der Formel

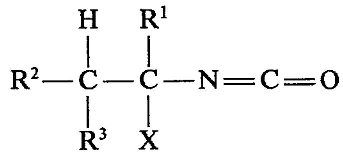

(Ia')

worin $R^1$, $R^2$ und $R^3$ gleich oder verschieden sein können und jeweils ein Wasserstoffatom, einen Alkylrest mit 1 bis 6 Kohlenstoffatomen oder einen Phenylrest bedeuten, das Restepaar $R^1$ und $R^2$ mit den benachbarten beiden Kohlenstoffatomen oder das Restepaar $R^2$ und $R^3$ mit dem benachbarten Kohlenstoffatom auch jeweils Glieder eines 5- oder 6gliedrigen, alicyclischen Ringes bilden können, wobei $R^1$, $R^2$ und $R^3$ insgesamt bis zu 8 Kohlenstoffatomen enthalten können, X ein Chloratom oder Bromatom bezeichnet, wobei X nur das Bromatom bezeichnet, wenn $R^1$ für einen Phenylrest und gleichzeitig $R^2$ und $R^3$ jeweils für ein Wasserstoffatom stehen.

**Claims**

1. A process for the preparation of a mixture of 1-monohalogenated isocyanates of the formula

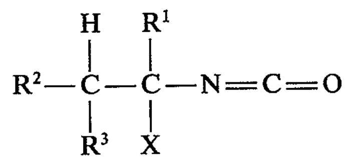

(Ia)

and 1,2-unsaturated isocyanates of the formula

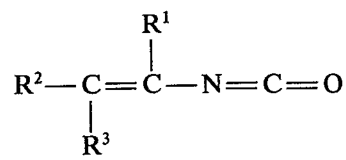

(Ib)

where $R^1$, $R^2$ and $R^3$ may be identical or different and each is hydrogen, alkyl of 1 to 6 carbon atoms or phenyl, or the pair of radicals $R^1$ and $R^2$ together with the two adjacent carbon atoms, or the pair of radicals $R^2$ and $R^3$ together with the adjacent carbon atom, can also form members of a 5-membered or 6-membered alicyclic ring, it being possible for $R^1$, $R^2$ and $R^3$ to contain a total of up to 8 carbon atoms, and X is chlorine or bromine, characterized in that

a 1) 1-monohalogenated carbamic halides of the formula

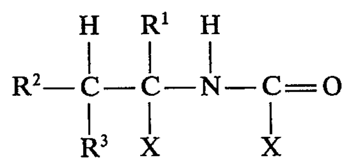

(II)

where $R^1$, $R^2$, $R^3$ and X have the above meanings, are reacted with a halogen-free isocyanate of the formula

$$R^4—N=C=O \qquad (III)$$

where $R^4$ is alkyl, cycloalkyl, aryl, aralkyl or alkylaryl, and/or
a 2) with a diisocyanate of the formula

$$O=C=N—R^5—N=C=O \qquad (IV)$$

where $R^5$ is alkylene, cycloalkylene, arylene, alkylarylene or arylalkylene, or

b 1) 1,2-unsaturated isocyanates I b are reacted with a 1-halogen-free carbamic acid halide of the formula

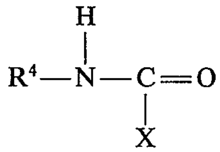

(V)

where $R^4$ and X have the above meanings and/or

b 2) with a bis-carbamic acid halide of the formula

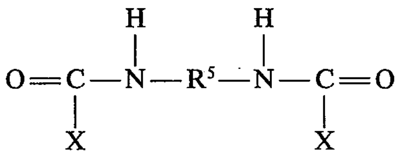

(VI)

where $R^5$ and X have the above meanings, and/or

b3) with a 1-monohalogenated carbamic acid halide II or

c1) 1-monohalogenated isocyanates Ia are reacted with a halogen-free isocyanate III and/or

c2) with a diisocyanate IV.

2. 1-Monohalogenated isocyanates of the formula

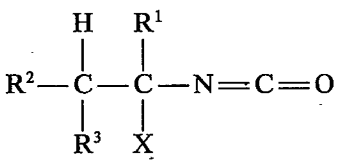

(Ia)

where $R^1$, $R^2$ and $R^3$ may be identical or different and each is hydrogen, alkyl of 1 to 6 carbon atoms or phenyl, or the pair of radicals $R^1$ and $R^2$ together with the two adjacent carbon atoms, or the pair of radicals $R^2$ and $R^3$ together with the adjacent carbon atom, can also form members of a 5-membered or 6-membered alicyclic ring, it being possible for $R^1$, $R^2$ and $R^3$ to contain a total of up to 8 carbon atoms, and X is chlorine or bromine, X only being bromine if $R^1$ is phenyl and at the same time $R^2$ and $R^3$ are each hydrogen.

**Revendications**

1. Procédé pour la préparation d'un mélange d'isocyanates 1-monohalogénés de la formule

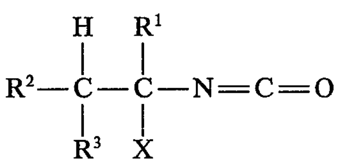

(Ia)

et d'isocyanates insaturés en position 1,2 de la formule

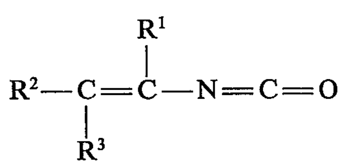

(Ib)

dans lesquelles $R^1$, $R^2$ et $R^3$, qui peuvent être identiques ou différents, désignent chacun un atome d'hydrogène, un radical alcoyle en $C_1$ à $C_6$ ou un groupe phényle, la paire de restes $R^1$ et $R^2$ et les deux atomes de carbone adjacents ou la paire de restes $R^2$ et $R^3$ et l'atome de carbone adjacent pouvant constituer des éléments d'un noyau alicyclique penta- ou hexagonal, le nombre total des atomes de carbone de $R^1$, $R^2$ et $R^3$ pouvant aller jusqu'à 8, et X représente un atome de chlore ou de brome,

caractérisé en ce que l'on fait réagir:

a 1) un halogénure de l'acide carbamique 1-monohalogéné de la formule

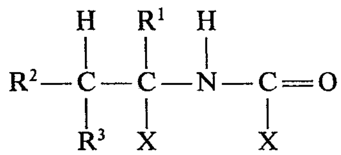

(II)

dans laquelle $R^1$, $R^2$ et $R^3$ possèdent la signification définie ci-dessus, avec un isocyanate exempt d'halogène de la formule

$$R^4—N=C=O \qquad (III)$$

dans laquelle $R^4$ désigne un groupe alcoyle, cycloalcoyle, aryle, aralcoyle ou alcoylaryle, et (ou)

a 2) avec un diisocyanate de la formule

$$O=C=N—R^5—N=C=O \qquad (IV)$$

dans laquelle $R^5$ représente un groupe alcoylène, cycloalcoylène, arylène, alcoylarylène ou arylalcoylène, ou

b 1) un isocyanate insaturé en position 1,2 de la formule Ib avec un halogénure de l'acide carbamique non halogéné en position 1 de la formule

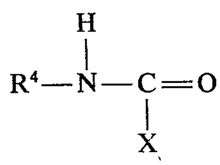

(V)

dans laquelle $R^4$ et X possèdent la signification définie plus haut, et (ou)

b 2) avec halogénure de bis-carbamyle de la formule

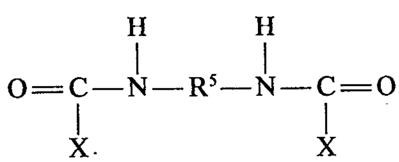

(VI)

dans laquelle $R^5$ et X possèdent la signification définie plus haut, et (ou)

b3) avec un halogénure d'acide carbamique 1-monohalogéné II ou

c1) un isocyanate 1-monohalogéné de la formula Ia avec un isocyanate non halogéné III et(ou)

c2) avec un diisocyanate IV.

2. Isocyanates 1-monohalogénés de la formule

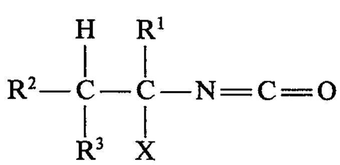

(Ia)

dans laquelle $R^1$, $R^2$ et $R^3$, qui peuvent être identiques ou différents, désignent chacun un atome d'hydrogène, un radical alcoyle en $C_1$ à $C_6$ ou un groupe phényle, la paire de restes $R^1$ et $R^2$ et les deux atomes de carbone adjacents ou la paire de restes $R^2$ et $R^3$ et l'atome de carbone adjacent pouvant constituer des éléments d'un noyau alicyclique penta- ou hexagonal, le nombre total des atomes de carbone de $R^1$, $R^2$ et $R^3$ pouvant aller jusqu'à 8, et X représente un atome de chlore ou un atome de brome, étant donné que X ne peut représenter qu'un atome de brome, lorsque $R^1$ est un groupe phényle et $R^2$ et $R^3$ désignent simultanément des atomes d'hydrogène.